# EUROPÄISCHE PATENTSCHRIFT

(11) **EP 0 974 061 B1**
(45) Veröffentlichungstag und Bekanntmachung des Hinweises auf die Patenterteilung: **02.01.2002**
(21) Anmeldenummer: 98919244.8
(22) Anmeldetag: 09.04.1998
(51) Int. Cl.: G01N 35/00, B65D 83/12

(54) **MAGAZIN ZUR BEVORRATUNG VON TESTELEMENTEN**
TEST ELEMENT STORAGE DEVICE
MAGASIN POUR STOCKAGE D'ELEMENTS D'ANALYSE

(30) Priorität: 11.04.1997 DE 19715031
(43) Veröffentlichungstag der Anmeldung: 26.01.2000
(73) Patentinhaber: Roche Diagnostics GmbH, 68298 Mannheim (DE)
(72) Erfinder: BOTTWEIN, Günter, D-68623 Lampertheim (DE); BÄR, Reinhard, D-68165 Mannheim (DE); LIST, Hans, D-64754 Hesseneck (DE); HEID, Friedrich, D-67122 Altrip (DE)
(86) Internationale Anmeldenummer: EP9802065
(87) Internationale Veröffentlichungsnummer: WO9847007

(56) Entgegenhaltungen:
- FR-A- 1 438 418
- US-A- 3 918 910
- US-A- 4 218 421
- US-A- 5 298 425

## Beschreibung

Die vorliegende Erfindung liegt im Gebiet der Bevorratung von Testelementen, wie sie zur Detektierung von Analyten in Probenflüssigkeiten verwendet werden. Derartige Testelemente werden insbesondere im Gebiet der Urinanalyse und im Gebiet der Blutzuckerbestimmung eingesetzt. Weiterhin werden Testelemente in großem Umfang in der Umweltanalytik eingesetzt. Ein weiteres Gebiet, dessen Bedeutung im Steigen begriffen ist, betrifft immunologische Teste, z.B. den Nachweis von Drogen, HCG und HIV in Blut oder Urin.

Zur Durchführung der Detektion eines Analyten bzw. der Analyse einer Probenflüssigkeit wird das Testelement mit einer Probe in Kontakt gebracht. Es kann dabei beispielsweise eine Oberfläche mit dem Testelement abgerieben werden. Der normale Anwendungsbereich von Testelementen liegt jedoch in der Analyse von Probenflüssigkeiten, wozu die Probenflüssigkeit entweder auf das Testelement aufgegeben wird (insbesondere Bestimmungen aus Blut, Serum, Liquor) oder das Testelement in die Probenflüssigkeit eingetaucht wird (insbesondere Urin).

Der überwiegende Teil von Testelementen muß gegen Feuchtigkeit geschützt werden, um eine Zersetzung von Reagenzien zu vermeiden. Dies gilt gleichermaßen für die sogenannten optischen Teststreifen, bei denen durch Reaktion eines Analyten mit der Probe eine Farbänderung hervorgerufen wird, wie auch für sogenannte Sensorelemente, bei denen eine durch die Probe hervorgerufene chemische Veränderung des Testelementes elektrochemisch ausgewertet wird. Weiterhin ist es normalerweise notwendig, Testelemente vor mechanischen Einflüssen und Verschmutzungen zu schützen, um verläßliche Analyseergebnisse sicherzustellen. Im Stand der Technik sind mehrere Arten zur Bevorratung von Testelementen bekannt. Bei der ersten Vorgehensweise befinden sich die Testelemente lose in einem Gefäß, beispielsweise in einer Röhre, die durch einen Stopfen oder dergleichen verschlossen werden kann. Weiterhin ist es bekannt, Testelemente einzeln in wasserdampfdichte Folien einzusiegeln. Mit den genannten Bevorratungsmethoden ist es jedoch schwierig, die Testelemente maschinell aus dem Vorratsgefäß bzw. der Siegelfolie zu entnehmen. Im Zusammenhang mit einer maschinellen Entnahme von Testelementen sind im Stand der Technik eine Reihe von Magazinen bekannt, wie sie beispielsweise in den Patenten US-5,102,624, US-5,154,889, US-3,918,910, US-4,911,344 und US-4,142,863 beschrieben sind. Bei diesem Typ von Magazin werden Testelemente verwendet, die übereinandergestapelt sind. Die in diesen US-Patenten beschriebenen Testelemente sind speziell für die Verwendung in einem Magazin angepaßt, d. h. sie besitzen eine gleichmäßige Dicke und sind ohne Verhaken oder Verklemmen gegeneinander verschiebbar. Der größte im Handel befindliche Anteil von Testelementen weist jedoch kein gleichmäßiges Höhenprofil auf und kann daher in den Magazinen des Standes der Technik nicht ohne weiteres verwendet werden. In der US-Patentschrift 3,918,910 wird ein Magazin beschrieben, das zur Bevorratung von Testelementen geeignet ist, bei denen auf einem Träger mehrere Nachweiszonen aufgebracht sind. Herstellungsbedingt stehen die Nachweiszonen über dem Träger über und verursachen so eine variierende Höhenstruktur des Testelementes. In den Figuren 5, 6, 7, 9 und 22 der US 3,918,910 ist ein Magazin gezeigt, das zur Bevorratung derartiger Testelemente geeignet ist. Bei diesem Magazin sind die Testelemente übereinandergestapelt, so daß jeweils Ober- und Unterseite von aufeinanderfolgenden Testelementen aneinander zu liegen kommen. Der Stapel von Testelementen wird durch Federelemente in Richtung eines Bereiches gedrückt, von wo aus die Entnahme eines Testelementes durch Herausschieben oder Herausziehen erfolgt. Experimentelle Untersuchungen haben gezeigt, daß derartige Magazine sehr fehleranfällig sind. Es kommt häufig zu Ausfällen, bei denen keines der Testelemente sicher durch die Entnahmeeinheit ergriffen werden kann oder fälschlicherweise gleich zwei Testelemente gemeinsam aus dem Magazin herausbefördert werden.

Im Stand der Technik sind weiterhin Vorrichtungen zum Vereinzeln von Teststreifen bekannt, mit denen aus einer ungeordneten Menge von Teststreifen einzelne Streifen entnommen werden können. In der EP-A-0 255 675 ist beispielsweise eine derartige Vorrichtung beschrieben. Eine weitere Vereinzelnungsvorrichtung ist in der US-5,298,425 offenbart, die auch eine Lagekorrektur der einzelnen Testelemente beschreibt. Vorrichtungen dieses Typs sind erfahrungsgemäß sehr störanfällig, insbesondere wenn die Testelmeente eine Biegung aufweisen.

Aufgabe der vorliegenden Erfindung war es, ein Magazin zur Verfügung zu stellen, das mit den im Handel weit verbreiteten Testelementen mit ungleichmäßigem Höhenprofil verläßlich arbeitet. Weiterhin war es Aufgabe der vorliegenden Erfindung, ein Magazin zur Verfügung zu stellen, mit dem eine maschinelle Entnahme möglich ist und das eine hohe Packungsdichte der Testelemente erzielt.

Eine weitere Aufgabe der vorliegenden Erfindung war es, ein System vorzuschlagen, das durch Zusammenwirken eines Magazins und einer Entnahmevorrichtung ein automatisiertes Zurverfügungstellen von Testelementen ermöglicht. Insbesondere war es die Aufgabe der Erfindung, die Fehleranfälligkeit bekannter Vorrichtungen des Standes der Technik zum Vereinzeln von Testelementen zu reduzieren.

Die Aufgabe der Erfindung wird durch eine Magazin zur Bevorratung von Testelementen gelöst, das mindestens ein Paar gegenüberliegenden Führungsnuten aufweist, in die die Testelemente so eingeschoben sind, daß sie direkt nebeneinander liegen und Kanten benachbarter Testelemente aneinanderstoßen.

Die vorliegende Erfindung umfaßt weiterhin ein System zum Zurverfügungstellen von Testelementen mit einem Magazin mit mindestens zwei gegenüberliegenden Führungsnuten, in die Testelemente so eingeschoben sind, daß sie in einer Fläche direkt nebeneinander liegen und Kanten benachbarter Testelemente aneinanderstoßen sowie einem Schieber, der an einem Ende der durch die Testelemente gebildeten Lage angreift und die Testelemente entlang der Nuten in Richtung auf das gegenüberliegende Ende der Lage verschiebt.

Im Rahmen der vorliegenden Erfindung wurde gefunden, daß die mit einem Magazin gemäß US-3,918,910 auftretenden Probleme hauptsächlich in einem Verkippen der Testelemente begründet sind. Unter Bezugnahme auf die Figur 5 der US-3,918,910 (siehe Figur 6 dieser Anmeldung) bedeutet dies, daß die Testelemente in eine Schräglage zur Platte (61) geraten oder sogar die Platte (61) selbst in eine Schieflage gerät. Insbesondere wurde dieses Problem beobachtet, wenn die verschiedenen Testzonen eines einzelnen Testelementes eine unterschiedliche Höhe aufweisen, was bei den im Handel befindlichen Testelementen mit mehreren Nachweiszonen häufig der Fall ist. Weiterhin wurde gefunden, daß die flexible Natur der Testelemente ein Verbiegen oder sogar Tordieren der Testelemente erlaubt, so daß ein sicheres Ergreifen eines derartigen Testelementes mit einer maschinellen Vorrichtung schwer möglich ist. Die vorliegende Erfindung schlägt daher ein Magazin vor, bei dem die Testelemente in Führungsnuten geführt werden und so angeordnet sind, daß Kanten benachbarter Testelemente direkt aneinander zu liegen kommen. Die vorliegende Erfindung nutzt die Tatsache, daß die Testelemente, bedingt durch ihre Herstellung, eine genau definierte und über die Länge des Teststreifens gleichbleibende Breite aufweisen. Werden die Testelemente, wie erfindungsgemäß vorgesehen, mit ihren beiden Enden in Nuten geführt und die Testelemente nebeneinander statt übereinander wie im Stand der Technik angeordnet, so können im Regelfall die Testelemente, zumindest aber die beiden Enden der Testelemente, definiert positioniert werden. Dementsprechend kann mit der vorliegenden Erfindung eine verläßliche maschinelle Entnahme von Testelementen aus dem Magazin realisiert werden. Insbesondere ist das Magazin der vorliegenden Erfindung zur Bevorratung von Testelementen geeignet, die flexibel sind und / oder ein ungleichmäßiges Höhenprofil besitzen. Im Rahmen der vorliegenden Anmeldung wird weiterhin ein System beschrieben, mit dem eine Entnahme von Testelementen aus dem erfindungsgemäßen Magazin möglich ist.

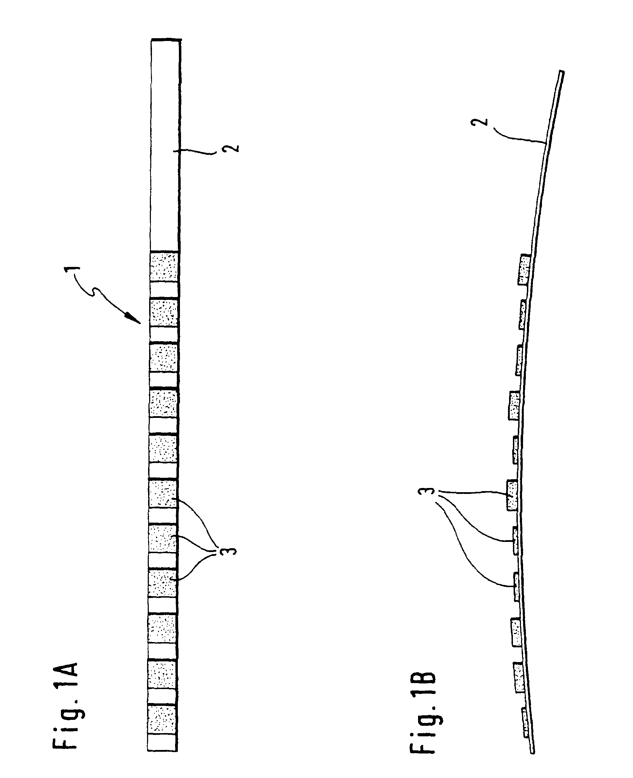

In den Figuren 1A und 1B der vorliegenden Anmeldung sind handelsübliche Testelemente dargestellt. Die Figur 1A zeigt ein Testelement in der Aufsicht. Das Testelement (1) besitzt einen Träger (2) auf dem mehrere Testzonen (3) aufgebracht sind. Das dargestellte rechteckige Testelement besitzt eine kürzere Seite, die im Folgenden als Breite bezeichnet wird, und eine längere Seite, die als Länge bezeichnet wird. In der Figur 1B ist eine Seitenansicht eines Testelementes dargestellt. Zu erkennen ist deutlich die unterschiedliche Höhe der Testzonen (3) sowie eine Verbiegung des Testelementes. Die Verbiegung des Testelementes kann sowohl durch die Eigenschaften des Trägers (2) bedingt sein als auch durch die Art der Aufbringung der Testzonen (3). Bei einer besonders günstigen Herstellungsweise von Testelementen werden die Testzonen auf den Träger aufgebracht und nachfolgend mit einem feinen Netz überzogen. Diese Vorgehensweise ist beispielsweise in der US-3,802,842 beschrieben. Durch die dem Netz innewohnende Neigung, sich zusammenzuziehen, findet eine Verbiegung des Testelementes statt. Weiterhin kann eine Verbiegung durch das Trägermaterial hervorgerufen werden. Als Trägermaterialien werden vorzugsweise Kunststoff-Folien eingesetzt, die bei der Herstellung der Testelemente in schmale Streifen geschnitten werden. Eine herstellungsbedingte Krümmung der Folie kann daher auch zu einer Krümmung des Testelementes führen.

Ein erfindungsgemäßes Magazin zur Bevorratung von Testelementen besitzt mindestens ein Paar gegenüberliegender Führungsnuten, in die die zu bevorratenden Testelemente eingeschoben werden. Die Führungsnuten führen ein Testelement jeweils an den beiden gegenüberliegenden Enden. Werden nacheinander mehrere Testelemente in eine Führungsnut eingeschoben, so kommen die Testelemente Seite an Seite zu liegen und die Längskanten der Träger benachbarter Testelemente berühren sich. Der Abstand der gegenuberliegenden Führungsnuten ist entsprechend der Länge der Testelemente gewählt. Der Abstand der Nutgründe (am weitesten auseinanderliegenden Bereiche der Nuten) entspricht vorzugsweise der Länge eines gestreckten Testelementes. Die Tiefe der Nuten und der Abstand der Nuten bestimmen die Länge des Testelementabschnittes, der durch die Nut geführt wird. Entspricht der Abstand der Nuten an ihrer tiefsten Stelle der Länge der Testelemente, so ist die geführte Länge der Testelemente gleich der Nutentiefe. Sind die Nuten jedoch weiter auseinander, so haben die Testelemente ein Spiel in ihrer Längsrichtung und die geführte Länge entspricht der Nutentiefe abzüglich dieses Spiels. Für ein einwandfreies Funktionieren des Magazins hat sich ein geringfügiges Spiel der Testelemente in ihrer Längsrichtung als günstig erwiesen. Dieses Spiel beträgt vorteilhaft 0,5 bis 20 % der Testelementlänge. Es hat sich herausgestellt, daß es in der Regel ausreichend ist, die Testelemente auf einer Länge von 0,3 cm zu führen. Vorzugsweise sind die Teststreifenabschnitte, die durch die Nuten geführt werden, 0,4 bis 1,0 cm lang. Erfindungsgemäß sollen auch solche Ausführungsformen umfaßt werden, bei denen die beiden Nuten jeweils so tief sind, daß sie eine Hälfte des Teststreifens aufnehmen und die Nuten weiterhin so miteinander verbunden sind, daß sich ein zusammenhängender Schlitz ergibt. Im Rahmen dieser Erfindung wird ein derartiger Schlitz als spezielle Ausgestaltung eines Nutenpaares verstanden. Ein derartiger Schlitz zur Führung der Testelemente ist günstig, da er dazu verwendet werden kann, die Krümmung der Testelemente zu unterdrücken, da die Testelemente auf ihrer vollen Länge geführt werden. Produktionstechnisch sind jedoch Magazinausführungen vorteilhafter, bei denen die Nuten eine Tiefe von weniger als 2 cm aufweisen.

Die Länge der Nuten bestimmt die Zahl der Testelemente, die aufgenommen werden können. Die Länge der Nuten unterliegt kaum Einschränkungen. Es sind jedoch Nutlängen zwischen 5 und 15 cm günstig, da sie zu leicht handhabbaren, kompakten Magazinen führen. Bei der Verwendung von Schlitzen (statt Nuten) zur Aufnahme der Testelemente ist deren Länge im wesentlichen werkzeugtechnisch limitiert, da es schwierig ist, Ausformwerkzeuge für große Schlitzlängen, beispielsweise oberhalb 10 cm, zur Verfügung zu stellen. Bei der Verwendung von Nuten entfällt jedoch diese Schwierigkeit, und man ist in der Nutenlänge kaum limitiert. In beiden Fällen, d. h. sowohl bei Verwendung von Nuten als auch von Schlitzen, muß sichergestellt werden, daß die Nuten bzw. die Schlitzenden im wesentlichen parallel verlaufen, um ein Verklemmen oder Herausfallen von Testelementen zu vermeiden.

Erfindungsgemäß ist es vorteilhaft, wenn das Magazin Paare von übereinander angeordneten Führungsnuten aufweist, da so die Lagerung von Testelementen in übereinander angeordneten Lagen möglich ist, was die Aufhahmekapazität des Magazins erhöht, von der Handhabung hat es sich als günstig erwiesen, wenn ein Magazin 8 bis 15 Lagen von Testelementen besitzt. Zur Bevorratung von 300 Testelementen ist es beispielsweise günstig, ein Magazin mit 12 Lagen zu verwenden, wobei sich in jeder Lage 25 Testelemente befinden.

Bereits eingangs wurde erwähnt, daß das Magazin neben einer Ordnung und einer Zurverfügungstellung von Testelementen die Aufgabe erfüllen kann, die Testelemente vor Feuchtigkeit sowie mechanischen Einwirkungen zu schützen. Das Magazin kann daher Wandungen besitzen, die den Innenraum gegenüber dem Außenraum abschließen. Zur Entnahme von Testelementen muß das Magazin eine oder mehrere Öffnungen aufweisen. Diese Öffnungen können durch einen mechanischen Verschluß, beispielsweise einen Klappdeckel oder eine aufgeklebte Siegelfolie, verschlossen sein. Weiterhin ist es vorteilhaft, das gesamte Magazin, auch wenn es verschlossen ist, zum Transport in eine wasserdampfdichte Umverpackung einzusiegeln.

Bei einer bevorzugten Ausführungsform des Magazins sind die Führungsnuten in die Seitenwände des Magazins integriert. Weiterhin können diese Seitenwände bereits während des Herstellungsprozesses durch weitere Wände, insbesondere Ober- und Unterwand, miteinander verbunden sein. Derartige Anordnungen können bevorzugt einteilig hergestellt werden. Insbesondere ist hierfür das Spritzgußverfahren geeignet. Werkzeugtechnisch kann es jedoch günstiger sein, die Wandungen des Magazins einzeln zu formen (vorteilhafterweise im Spritzgußverfahren). Da die Nuten einer Magazinseite durch eine Werkzeughälfte geformt werden, haben die einzelnen Nuten zueinander nur geringe Toleranzen. Wenn die Magazinteile einzeln geformt werden, so sind vorteilhaft jeweils die Seitenwände, Ober- und Unterseite, Vorder- und Rückseite identisch, so daß jeweils gleiche Werkzeuge verwendet werden können.

Vorteilhaft ist es jedoch auch, in einem Spritzgußprozeß die Wandungen einzeln so zu formen, daß sie durch Filmscharniere miteinander verbunden sind. Ein derartiges Gebilde (Faltschachtel) kann nach der Ausformung durch Zusammenrasten der einzelnen Wände in ein Magazin überführt werden.

Bei einer weiteren Ausführungsform wird die Kassette aus n+1 Spritzgußteilen zusammengesteckt, wobei n die Anzahl der Streifenlagen ist. Alle Teile sind gleich (Wiederholteile). Es sind flache Rahmen, die in einem Spritzgußprozeß ohne Schieber entformt werden können. Werden diese flach auf flach zusammengesteckt (geklipst, ultraschallgeschweißt oder geklebt), so bilden je zwei Stück die einander gegenüberliegenden Nuten für eine Lage Streifen. Günstig ist es, wenn die Rahmen zwei Ausnehmungen besitzen. Aus der jeweils kleineren Ausnehmung der Rahmen formt sich dabei ein oben und unten offener Schacht, in den ein Beutel mit Trockenmittel gelegt werden kann. Dieser kann beispielsweise durch eine Papierbanderole am Herausfallen gehindert werden.

Die Nutbreite und damit die Teilung der Streifenlagen ist bei dieser Ausführungsform geringfügig unpräziser, da sich die Fügetoleranzen auswirken.

Wie bereits vorangehend erwähnt, ist es zur Entnahme von Testelementen erforderlich, daß das Magazin Öffnungen aufweist. Es hat sich dabei als vorteilhaft erwiesen, wenn eine oder mehrere Öffnungen in eine Seitenwand des Magazins integriert sind. Eine derartige Öffnung befindet sich auf Höhe einer Nut, so daß ein Testelement, das sich in der Nut benachbart zu der Öffnung befindet, durch Verschieben entlang seiner Längsachse aus dem Magazin entnommen werden kann. Die Öffnung weist hierzu vorzugsweise einen rechteckigen Querschnitt auf, dessen Höhe um 10 bis 50 % größer ist als die maximale Höhe der Testelemente und dessen Breite um 10 bis 50 % größer ist als die Breite der Testelemente. Bei Einsatz mehrerer Paare von Nuten in einem Magazin besitzt vorzugsweise jedes Nutenpaar eine derartige Entnahmeöffnung. Weiterhin ist es vorteilhaft, wenn jedes der Nutenpaare eine weitere Öffnung in der gegenüberliegenden Seitenwand aufweist, durch die ein Schieber in das Magazin eindringen kann, um ein Testelement herauszustoßen. Die vorstehend genannten Öffnungen können bei einem Magazin, das in den Handel gebracht wird, durch Siegelfolien zumindest teilweise verschlossen sein. Diese Siegelfolien können vor der Benutzung abgezogen werden. Vorteilhaft ist es jedoch, wenn die Siegelfolien durch den Schieber bzw. ein austretendes Testelement durchstochen werden.

Bei einer weiteren, besonders einfachen, Ausführungsform des Magazins erfolgt die Entnahme von Testelementen durch Verschieben der Testelemente über das Nutende (in Längsrichtung der Nuten) hinaus, d. h. es findet eine Verschiebung quer zur Längsachse der Testelemente statt. Bei dieser Ausführungsform sind die Nuten eines Nutenpaares an einer Seite geöffnet, so daß ein Hinausschieben von Testelementen über das Nutende möglich ist. Das Magazin besitzt also eine Stirnfläche, die quer zu den Seitenwänden des Magazins verläuft und in der sich pro Nutenpaar eine Öffnung zum Austritt von Testelementen befindet. Diese Austrittsöffnungen haben vorzugsweise einen rechteckigen Querschnitt, dessen Breite um 10 bis 30 % größer ist als die maximale Höhe der Testelemente und dessen Länge um 0,5 bis 20 % größer ist als die Teststreifenlänge. Bei einer besonders einfach herzustellenden Variante dieser Ausführungsform besitzt das Magazin keine Stirnwand und die Stirnseite ist zum Transport des Magazins lediglich mit einer Siegelfolie verschlossen. Die Siegelfolie kann dazu dienen, die Stirnwand gegen ein Eindringen von Feuchte zu verschließen. Wird der Feuchtigkeitsschutz jedoch auf andere Weise realisiert (z.B. durch eine Umverpackung), so kann auf die Siegelfolie gegebenenfalls verzichtet werden. Als besonders wirkungsvoll hat es sich jedoch herausgestellt, die Bereiche der Stirnseite, in denen sich die Nutenenden befinden, mit einer Folie zu überkleben. Die Testelemente werden so einerseits am Herausfallen gehindert, andererseits kann die Folie von den Testelementen durchstoßen werden. Ein besonderer Vorteil dieser Ausführungsform liegt darin, daß die Folie nur jeweils im Bereich der Lage geöffnet wird, aus der eine Entnahme erfolgt. Die übrigen (noch vollständigen) Lagen von Testelementen sind weiterhin durch die Folie geschützt, so daß beim Verkippen des Magazins keine Testelemente herausfallen können.

Zur sukzessiven Entnahme von Testelementen aus dem Magazin ist es erforderlich, daß die Testelemente entweder in eine Entnahmeposition transportiert werden können oder direkt aus dem Magazin ausgeworfen werden. Im einfachsten Fall wird hierzu das Magazin so angeordnet, daß die Nuten senkrecht stehen und die Testelemente in den Nuten durch die Schwerkraft nach unten transportiert werden. An dem unteren Ende des Magazins befindet sich in diesem Fall eine Entnahmeposition oder das Magazin ist geöffnet, so daß die Testelemente direkt aus dem Magazin austreten können. Im letzteren Fall ist es erforderlich, daß ein Verschluß vorhanden ist, der ein unkontrolliertes Herausfallen von Testelementen verhindert und eine sukzessive Entnahme ermöglicht.

Vorzugsweise enthält das Magazin in seinem Inneren ein oder mehrere Federelemente, die das Ensemble von Testelementen, das sich in einem Nutenpaar befindet, gemeinsam in Richtung auf eine Entnahmeposition transportieren. Derartige Federelemente können beispielsweise Spiralfedern sein, die an dem der Entnahmeposition abgewandten Ende jeder Nut angeordnet sind. Federelemente stabilisieren weiterhin die Anordnung der Testelemente, auch wenn das Magazin nicht vollständig gefüllt ist. Es wird somit vermieden, daß sich die Testelemente quer stellen oder in einer anderen Weise in Unordnung geraten, was die Zuverlässigkeit eines Magazins stark herabsetzen würde.

Bei einer besonders bevorzugten Ausführungsform des Magazins besitzt dieses mindestens eine Ausnehmung in der Oberseite oder Unterseite, durch die ein Schieber in das Magazin eingeführt werden kann. Mit einem solchen Schieber ist es möglich, den Transport der Testelemente von außerhalb vorzunehmen. Der Schieber kann an der Außenseite des Magazins befestigt sein, so daß er von einem Benutzer manuell verschoben werden kann. Vorzugsweise gehört der Schieber jedoch zu einem Analysegerät, das mit Testelementen arbeitet. Der Schieber wird in diesem Fall durch eine zum Analysegerät gehörige Antriebseinheit gesteuert, so daß die Entnahme von Testelementen durch das Analysegerät gesteuert werden kann. Hierdurch kann erreicht werden, daß Testelemente erst dann aus dem schützenden Magazin entnommen werden, wenn sie vom Analysegerät benötigt werden.

Eine Einheit, beinhaltend ein Magazin sowie einen Schieber, der zur Entnahme von Testelementen dient, bildet ein System zum Zurverfügungstellen von Testelementen, das ebenfalls Gegenstand dieser Erfindung ist. Zu diesem System gehört ein Magazin mit mindestens zwei gegenüberliegenden Führungsnuten, in die die Testelemente so eingeschoben sind, daß sie in einer Fläche direkt nebeneinander liegen, und die Kanten benachbarter Testelemente aneinanderstoßen. Weiterhin gehört zum System ein Schieber, der an einem Ende der durch die Testelemente gebildeten Fläche angreift und die Testelemente quer zu ihrer Längsachse in Richtung auf das gegenüberliegende Ende der Fläche verschiebt. Wie bereits vorstehend beschrieben, greift der genannte Schieber vorzugsweise durch eine Ausnehmung in einer Magazinwand in das Magazin ein. Die hierzu benötigte Ausnehmung wird möglichst klein gehalten, um ein Eindringen von Feuchte in das Magazin zu minimieren. Vorzugsweise ist die Ausnehmung ein Schlitz, der parallel zu den Nuten in der Ober- oder Unterseite des Magazins verläuft. Der Schlitz kann an den gegenüberliegenden Längsseiten Gummilippen besitzen, die in der Ruhelage aneinander anliegen und so ein Eindringen von Feuchte weitestgehend verhindern. Zur Entnahme wird ein Schieber zwischen den Gummilippen hindurchgeführt und entlang des Schlitzes verschoben. Aufgrund der elastischen Eigenschaft der Gummilippen entstehen nur im Durchtrittsbereich des Schiebers durch die Gummilippen kleinere Undichtigkeiten und der größte Teil des Schlitzes ist hinreichend gegen Feuchte verschlossen.

Zur Entnahme von Testelementen wird der Schieber inkrementell um ein Wegstück verschoben, daß im wesentlichen der Breite eines Testelementes entspricht, so daß die Testelemente nacheinander in eine Entnahmeposition gebracht oder aber direkt aus dem Magazin ausgeworfen werden. Die Antriebseinheit für den Schieber kann beispielsweise eine Mutter sein, die sich auf einer Gewindestange befindet. Die Gewindestange kann über einen Schrittmotor gedreht werden, der seinerseits von einer Steuereinheit angesteuert wird. Wie bereits vorangehend erwähnt, ist es vorteilhaft, wenn die Steuereinheit mit der Steuereinheit eines Analysegerätes verbunden ist, so daß der Entnahmezeitpunkt geeignet gesteuert werden kann. An der genannten Mutter kann der Schieber, beispielsweise in Form eines Metallstiftes, direkt befestigt sein. Eine Drehung der Gewindestange bewirkt einen linearen Vorschub des Stiftes, der zur Verschiebung der Testelemente eingesetzt wird.

Bei Verwendung eines Magazins mit mehreren Nutenpaaren ist es vorteilhaft, wenn die Eindringtiefe des Schiebers in das Magazin gesteuert werden kann. Bei einer vorteilhaften Vorgehensweise dringt der Schieber zunächst so tief in das Magazin ein, daß er sich auf Höhe einer ersten Lage (Ebene) von Testelementen befindet, und transportiert das in dieser Ebene befindliche Ensemble von Testelementen, so daß die einzelnen Testelemente sukzessive in eine Entnahmeposition gelangen. Nach vollständiger Entleerung dieser Lage fährt der Schieber zurück, die Eindringtiefe wird vergrößert, so daß er sich auf Höhe einer zweiten Lage von Testelementen befindet und der Entnahmevorgang wird wiederholt. Entsprechend können weitere Lagen von Testelementen entleert werden.

Zur sukzessiven Entnahmen von Testelementen aus dem Magazin ist es wichtig, das die Verschiebung der Testelemente in die Entnahmeposition innerhalb des Magazins sowie die Entnahme der Testelemente aus dieser Position in aufeinander abgestimmter Weise erfolgen. Die Entnahme von Testelementen aus der Entnahmeposition kann durch einen zweiten Schieber erfolgen, der das jeweilige in der Entnahmeposition befindliche Testelement entlang seiner Längsachse verschiebt und es somit aus dem Magazin herausstößt. Weiterhin kann die Entnahme erfolgen, indem ein Greifer in das Magazin eingeführt wird und ein Testelement aus der Entnahmeposition herauszieht. Es sind auch Kombinationen der beiden Entnahmevorgänge möglich, bei denen das Testelement zunächst durch einen Schieber ein Stück entlang seiner Längsachse verschoben wird, so daß das Testelement besser von einer Handhabungseinheit ergriffen werden kann.

Ein Entnahmezyklus für Testelemente beinhaltet die folgenden Schritte:
- Entnahme eines Testelementes aus einer Entnahmeposition des Magazins (kann durch Herausschieben, Herausziehen oder eine Kombination beider Vorgänge erfolgen)
- Verschieben des in einer Ebene befindlichen Ensemble von Testelementen, so daß ein neues in die Entnahmeposition befördert wird

Im Rahmen der vorliegenden Erfindung wird weiterhin ein System mit einem erfindungsgemäßen Magazin sowie einem Schieber zum Verschieben der Teststreifenlagen und einer Walze mit mindestens einer Nut, in die ein Testelement hineingeschoben und durch Drehung der Walze transportiert werden kann, beschrieben. Vorzugsweise befindet sich das Magazin sowie ein Bereich der Walze in einem feuchtigkeitsdicht verschlossenen Gehäuse, so daß die in dem Magazin befindlichen Testelemente nicht durch Feuchtigkeit zerstört werden. Durch eine koordinierte Betätigung des Schiebers und der Walze können die Testelemente sukzessive aus dem Gehäuse heraustransportiert werden.

Die vorliegende Erfindung wird anhand der folgenden Figuren näher erläutert:
- Fig. 1A:: Testelement in Aufsicht
- Fig. 1B:: Seitenansicht eines Testelementes
- Fig. 2:: Schnitt durch ein Magazin entlang einer Ebene von Testelementen
- Fig. 3:: Schnitt durch ein Magazin entlang Schnittlinie A' in Figur 2
- Fig. 4:: Schnitt durch ein Magazin entlang der Linie B' in Figur 2
- Fig. 5:: Ausschnittsvergrößerung des Bereiches X in Figur 4
- Fig. 6:: Stand der Technik (US-3,918,910)
- Fig. 7:: Aus zusammengesteckten Rahmen aufgebautes Magazin
- Fig. 8:: System zum Zurverfügungstellen von Testelementen
- Fig. 9:: Walze mit Nut zur Aufnahme von Testelementen
- Fig. 10:: System zur Entnahme von Testelementen aus der Walze
- Fig. 11:: System zum Zurverfügungstellen von Testelementen mit Nachfülleinrichtungen

Figur 1 zeigt ein Testelement in Aufsicht. Es sind weiterhin der Träger (2) und die Testzonen (3) zu erkennen. In der Figur 1 B ist ein weiteres Testelement in Seitenansicht dargestellt. Insbesondere zeigt Figur 1 B die unterschiedliche Höhe der Testzonen (3) sowie die Verbiegung des Trägers (2).

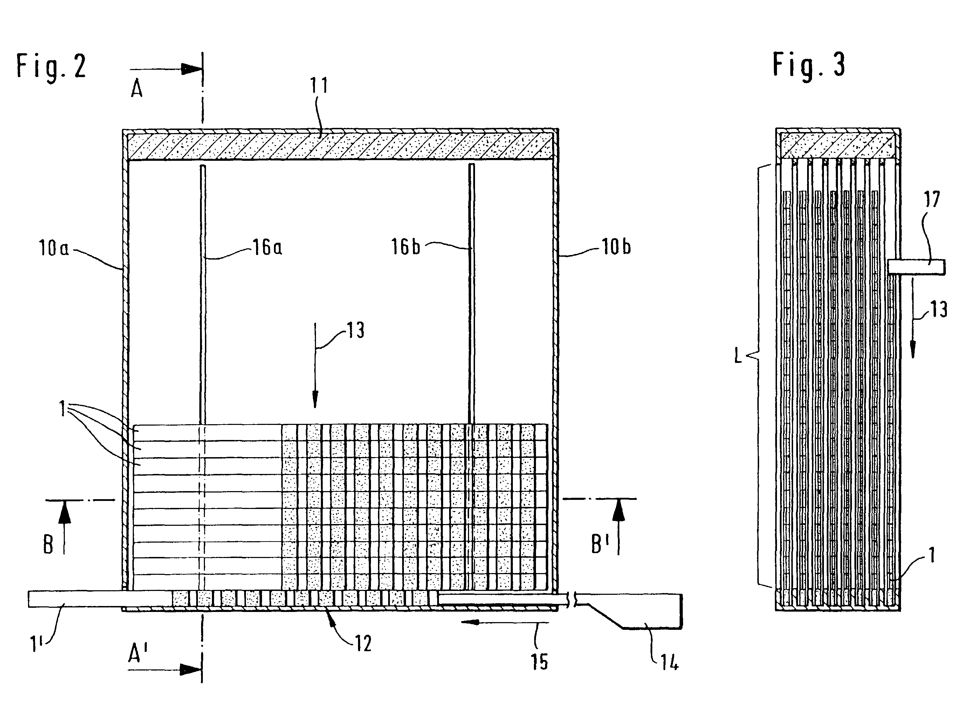

In der Figur 2 ist ein Magazin entlang einer Lage (Ebene) von Testelementen (1) dargestellt. Im Magazin sind die Testelemente gleichsinnig Seite an Seite angeordnet. In den Seitenwänden (10a) und (10b) befinden sich die Nuten zur Aufnahme der Testelemente. An seiner Rückseite besitzt das Magazin eine Trockenmittelkammer (11), die dazu dient, in das Magazin eingedrungene Feuchte zu absorbieren. Als Trockenmittel sind die im Stand der Technik bekannten Substanzen, z. B. Kieselgele oder Molekularsiebe, verwendbar. Die Trockenmittelkammer (11) verschließt die Rückseite des Magazins so, daß keine Öffnungen zurückbleiben, um ein Eindringen von Feuchte zu verhindern.

In der Figur 2 ist eine bereits halb entleerte Lage von Testelementen dargestellt. Zur Entleerung werden die Testelemente von der Rückseite des Magazins in Richtung auf die Vorderseite (12) bewegt, wie dies durch den Pfeil (13) angedeutet ist. Das der Vorderseite nächstliegende Testelement (1') befindet sich in der Entnahmeposition. Zur Entnahme wird das Testelement (1') durch einen Ausstoßschieber (14) in der durch den Pfeil (15) dargestellten Richtung aus dem Magazin herausgestoßen. Hierzu besitzt das Magazin in der rechten Seitenfläche (10b) eine Öffnung, durch die der Ausstoßschieber (14) in das Magazin eindringen kann. Weiterhin besitzt das Magazin in der linken Seitenfläche (10a) eine Öffnung, durch die das Testelement (1') aus dem Magazin austreten kann.

In der Figur 2 sind weiterhin die Schlitze (16a) und (16b) dargestellt, obwohl sich diese in der Oberseite des Magazins befinden und nicht in der Ebene der Testelemente liegen Durch die Schlitze (16a) und (16b) können Schieber eingreifen, die das Ensemble von Testelementen in Richtung des Pfeiles (13) bewegen.

Figur 3 zeigt einen Schnitt durch das Magazin der Figur 2 entlang der Linie (A - A'). In Figur 3 ist der Schieber (17) zum Vorschieben der Testelemente in Richtung der Entnahmeposition dargestellt. Weiterhin ist aus Figur 3 die Nutenlänge (L) zu entnehmen.

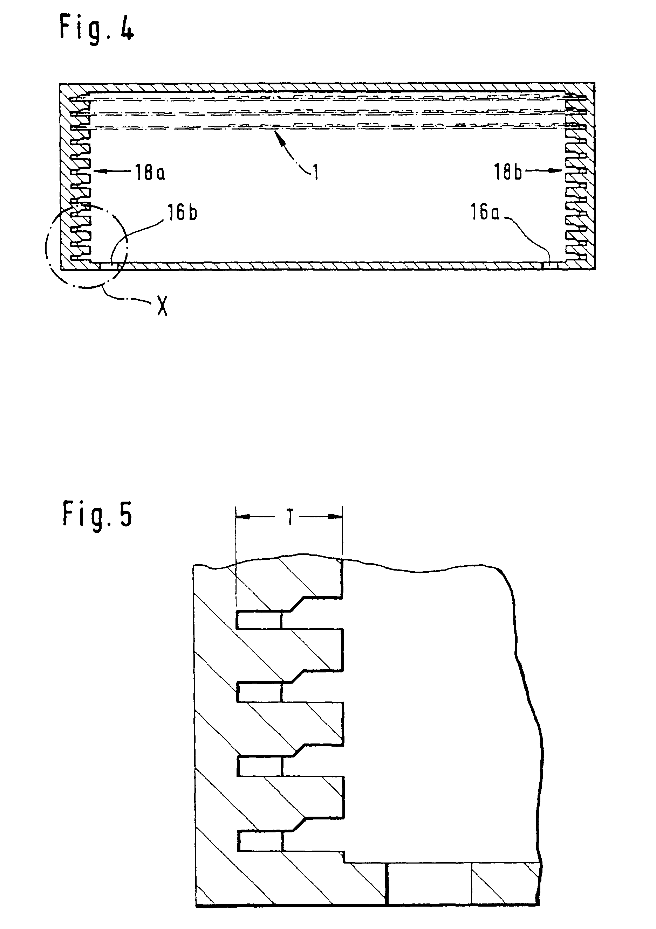

Figur 4 zeigt eine Darstellung des Magazins aus Figur 2 entlang der Schnittlinie (B - B'). In der Figur 4 sind die Paare von Nuten (18a, 18b) zu erkennen, in denen die Testelemente geführt werden. Zu einem Nutenpaar gehören jeweils zwei sich gegenüberliegenden Nuten Das in der Figur 4 dargestellte Magazin besitzt 12 solcher Nutenpaare, so daß Testelemente in 12 Ebenen bzw. Lagen bevorratet werden können.

Die Figur 5 zeigt eine Ausschnittsvergrößerung (5fach) des Bereiches (X) in Figur 4, in dem die Struktur der hier verwendeten Nuten genauer zu erkennen ist. Die dargestellten Nuten weisen eine Tiefe (T) auf, in die die Testelementenden eingeführt werden. Es ist aus der Figur 5 zu erkennen, daß jede der Nuten einen schmaleren und einen breiteren Teil besitzt. Dies ist vorteilhaft, wenn die Testzonen auf dem Träger nah an ein Ende des Testelementes heranreichen. In diesem Fall kann vorteilhaft eine Nut mit zwei Dickenbereichen verwendet werden, so daß das äußerste Testelementende, welches nicht von einer Testzone überdeckt ist, durch den schmaleren Bereich der Nut geführt wird, und weiterhin ein benachbarter Bereich, in dem sich eine Testzone befindet, durch den breiteren Bereich der Nut geführt werden kann.

Der Abstand der in Figur 5 dargestellten Nuten beträgt ca. 2 mm. Ein derartig geringer Abstand kann trotz einer Verbiegung der Testelemente realisiert werden, ohne daß die Gefahr eines Verhakens benachbarter Ebenen von Testelementen besteht. Hierzu ist es vorteilhaft, die Testelement in den Ebenen gleichsinnig anzuordnen, so daß die Oberseite eines Testelements in einer Lage benachbart zur Unterseite eines Testelements in der nächst höheren Lage zu liegen kommt. Da eine Verbiegung der Testelemente normalerweise für jede Charge in der gleichen Richtung auftritt, besitzen die Lagen der Testelemente die gleiche Wölbungsrichtung und ein ausreichender Abstand zwischen den Lagen bleibt gewahrt.

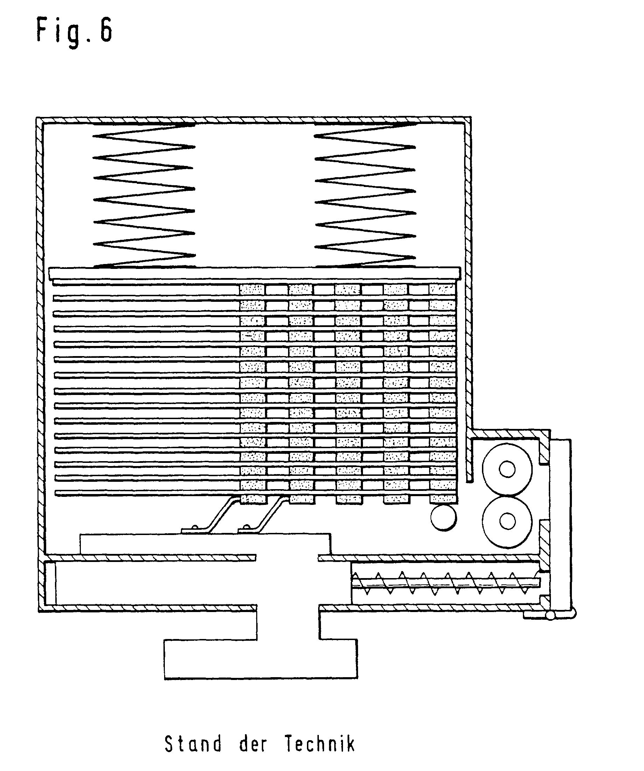

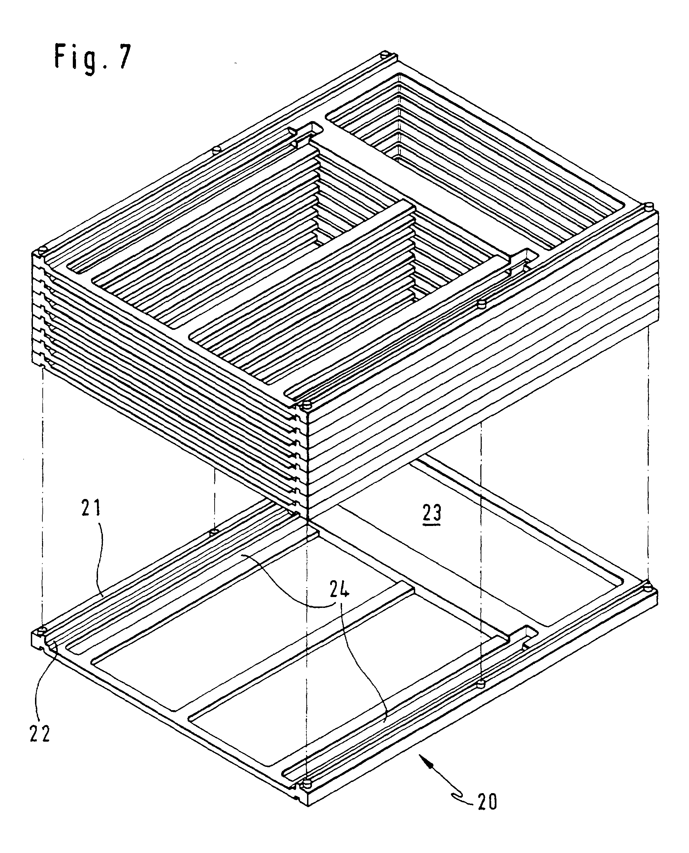

Figur 7 zeigt eine Explosionszeichnung eines Magazins, das aus einzelnen Rahmen (20) zusammengesteckt oder zusammengeklipst ist. Die Rahmen sind im dargestellten Beispiel so beschaffen, daß der seitliche Rand (21) gegenüber einem Absatz (22) erhöht ist. Die Unterseite der Rahmen ist im Bereich des Randes (21) und des Absatzes (22) im wesentlichen planar, so daß sich beim Übereinanderstapeln von zwei oder mehr Rahmen eine Nut ergibt, die durch den Absatz (22) und der darüberliegenden Bodenfläche des nächsten Rahmens gebildet wird. Die Rahmen besitzen einen vorderen Teil, in dem sich die Nuten befinden, sowie eine rechteckige Ausnehmung (23). Diese führt im zusammengesteckten Magazin zu einem Hohlraum, der mit einem Trockenmittel bzw. einem Trockenmittelbehältnis gefüllt werden kann. Die Rahmen (20) können weiterhin Schlitze (24) (im dargestellten Beispiel zwei) besitzen, in die ein Schieber zum Verschieben der Testelemente eingreifen kann.

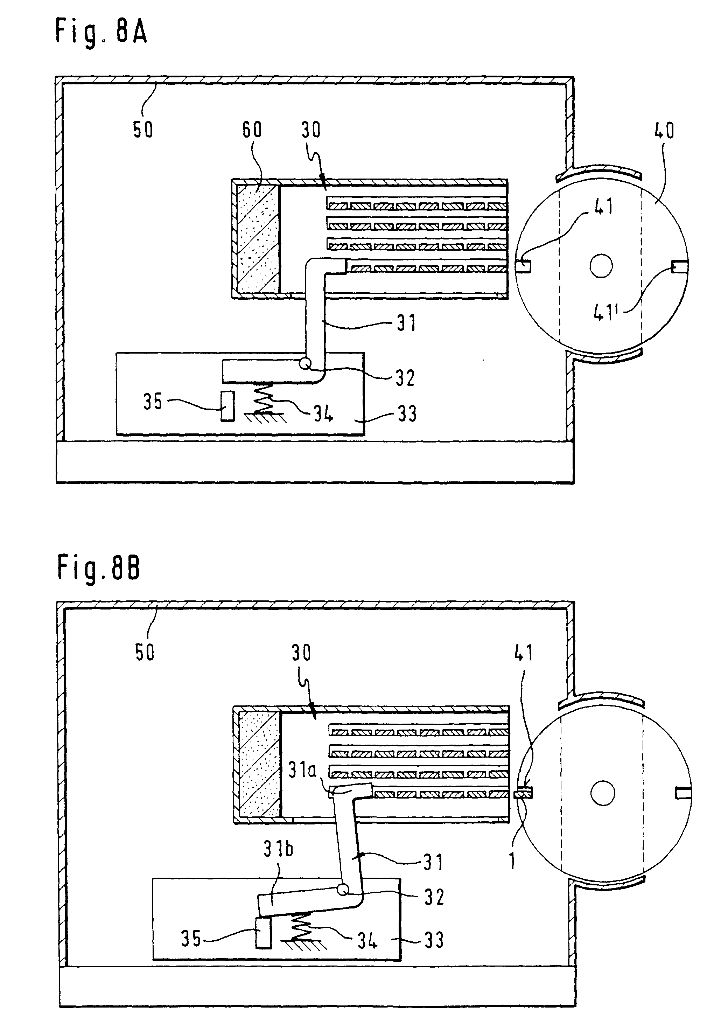

In der Figur 8 ist ein System zum Zurverfügungstellen von Testelementen dargestellt. Die Figuren 8A bis 8E zeigen die Schritte, die bei der Entnahme eines Testelementes durchlaufen werden. Das in der Figur 8A dargestellte System besitzt ein Magazin (30), in dem mehrere Lagen von Kante an Kante liegenden Testelementen angeordnet sind. Dieses Magazin entspricht im wesentlichen dem in der Figur 3 dargestellten. Insbesondere sind die Nuten des Magazins an der dem Trockenmittel (60) abgewandten Seite offen, so daß Testelemente durch Verschiebung einer Lage von Testelemente sukzessive aus dem Magazin herausbefördert werden können. An der Unterseite weist das Magazin einen oder mehrere Schlitz(e) auf, durch den der/die Schieber (31) in das Magazin eingreifen kann. Der Schieber ist seinerseits über eine Achse (32) drehbar mit einem verfahrbaren Schlitten (33) verbunden. Zur Entnahme von Testelementen wird das Magazin (30) in eine Höhe verfahren, in der eine Lage von Testelementen auf Höhe des vorderen Endes (31a) des Schieber zu liegen kommt. Nunmehr wird der Schieber in Richtung auf die Lage von Testelementen verschoben, wozu der Schlitten verfahren wird. Der Schieber greift wie in Figur 8A dargestellt, an der Lage von Testelementen an und verschiebt die gesamte Lage, wodurch die Testelemente in ihrer Querrichtung auf das geöffnete Ende des Magazins zubewegt werden. Gegenüber der Öffnung des Magazins ist eine drehbar gelagerte Walze angeordnet, die mindestens eine Nut zur Aufnahme eines Testelementes besitzt. Vorzugsweise besitzt die Walze zwei Nuten von im wesentlichen gleicher Geometrie. Zur Entnahme eines Testelementes wird die Walze (40) so gedreht, daß sich die mindestens eine Nut (41) auf Höhe der vom Schieber bewegten Teststreifenlage befindet. Durch Verschiebung des Schiebers wird das dem Schieber am weitesten abgewandte Testelement der Lage in die Nut (41) hineingeschoben. Figur 8B zeigt einen Zustand, in dem sich das genannte Testelement bereits in der Nut befindet. Durch die Bewegung des Schlitten (33) wird das Testelement bis an das Ende der Nut (41) herangeschoben und eine Weiterbewegung des Schlittens (33) führt, da die Lage der Kante an Kante geordneten Testelemente nicht beliebig komprimiert werden kann, zu einer Drehung des Schiebers (31) um die Achse (32). Vorzugsweise wird der Schieber durch eine Feder (34) in seiner Ruheposition gehalten und ein Herausbewegen aus der Ruhelage durch Drehung um die Achse wird mit einem Sensor (35) detektiert. Dieser Sensor kann beispielsweise eine Lichtschranke sein, deren Lichtweg durch Eintauchen des hinteren Endes (31b) des Schiebers unterbrochen wird. Diese Unterbrechung kann mit einer Steuerungsvorrichtung detektiert und die Verschiebung des Schlittens (33) in Richtung auf die Walze (40) gestoppt werden. Mit dieser Vorrichtung kann demnach ein Testelement aus dem Magazin sicher und vollständig in die Nut geschoben werden, ohne daß die Breite des Testelementes bekannt zu sein braucht. Somit ist die Vorrichtung weitestgehend unabhängig von Schwankungen der Testelementbreite, die produktionstechnisch quasi unvermeidbar ist. Ausgehend von der in Figur 8B dargestellten Position wird der Schlitten in die entgegengesetzte Richtung verfahren, wodurch der Druck von der Lage der Testelemente weggenommen wird und der Schieber in seine Ruheposition bezüglich der Rotation um die Achse (32) zurückkehrt. Das nunmehr in der Walze befindliche Testelement kann durch eine Drehung der Walze abtransportiert werden. Vorzugsweise befindet sich das Magazin sowie der zur Entnahme eines Testelementes dienende Teil der Walze innerhalb eines feuchtigkeitsdichten Gehäuses (50). Hierdurch kann erreicht werden, daß die in dem Magazin befindlichen Testelemente nicht durch Feuchtigkeit zersetzt werden. Dies wird vorzugsweise zusätzlich durch ein zum Magazin gehörendes Trockenmittel (60) sichergestellt. Da sich die Nut zur Aufnahme eines Testelementes sowohl innerhalb des Gehäuses (50) als auch außerhalb davon befinden kann, müssen besondere Vorkehrungen getroffen werden, um den Bereich der Walze gegenüber einem Eindringen von Feuchte abzudichten. Dies kann beispielsweise durch Gummilippen oder dergleichen erfolgen, die dicht an der Walze anliegen und auch für eine Feuchtigkeitsabdichtung sorgen, wenn die Walze gedreht wird. Da die Nut beladen mit einem Testelement jedoch unter einer derartigen Gummilippe hindurchgedreht werden muß, hat sich der in Figur 8 dargestellte Aufbau zur Vermeidung eines Eindringens von Feuchte als besser geeignet herausgestellt. Das Gehäuse (50) besitzt Schutzteile (50a, 50b), die so gegenüber der Außenumfangsfläche der Walze (40) angeordnet sind, daß die Walze in diesen Bereichen möglichst dicht umgeben ist. Zwischen den Schutzteilen (50a, 50b) und der Walze (40) bestehen Spalte, die ein leichtgängiges Drehen der Walze ermöglichen und gewährleisten, daß auch Testelemente, die über die Außenkontur der Walze überstehen, zerstörungsfrei transportiert werden können. Der Spalt zwischen den Schutzteilen und der Walze stellt eine Kriechstrecke für Feuchte dar, durch die eine gewisse Menge an Feuchte eindringen kann. Diese Menge ist jedoch so gering ist, daß sie zu keiner rapiden Zersetzung der Testelemente Anlaß gibt.

In der Figur 8D ist zu erkennen, wie das in der Nut befindliche Testelement durch Drehen der Walze durch den Spalt zwischen dem Schutzteil (50a) und der Walze transportiert wird. In den Figuren 8C und 8D ist zu erkennen, daß das Testelement geringfügig über die Außenkontur der Walze übersteht. Dies entspricht einer bevorzugten Ausgestaltung, bei der die Tiefe der Nut geringer ist als die kleinste zu erwartende Testelementbreite. Hierdurch wird sichergestellt, daß jedes Testelement zumindest geringfügig über die Außenkontur der Walze übersteht und somit kein nachfolgendes Testelement zusammen mit dem bereits in der Nut befindlichen Testelement in die Nut eindringt. Eine derartige Situation ist zu vermeiden, da ein Drehen der Walze zu einem Systemausfall infolge Verklemmens oder ähnlichem führen würde. In Figur 8C ist ebenfalls zu erkennen, daß der Spalt zwischen dem Schutzteil (50a) und der Walze größer ist als der Spalt zwischen dem Schutzteil (50b) und der Walze. Um ein Herausfallen von Testelementen aus der Nut zu verhindern, wird das Testelement durch den oberen Spalt transportiert. Der Spalt zwischen dem Schutzteil (50b) und der Walze kann daher so klein gemacht werden, wie dies die Fertigungstoleranzen erlauben.

Im Regelfall erfolgt zur Entnahme eines Testelementes aus dem Magazin eine 180 °- Drehung der Walze, mit der das Testelement in die in Figur 8E dargestellte Position befördert wird, aus der es mit einem Greifarm oder dergleichen abgeholt werden kann.

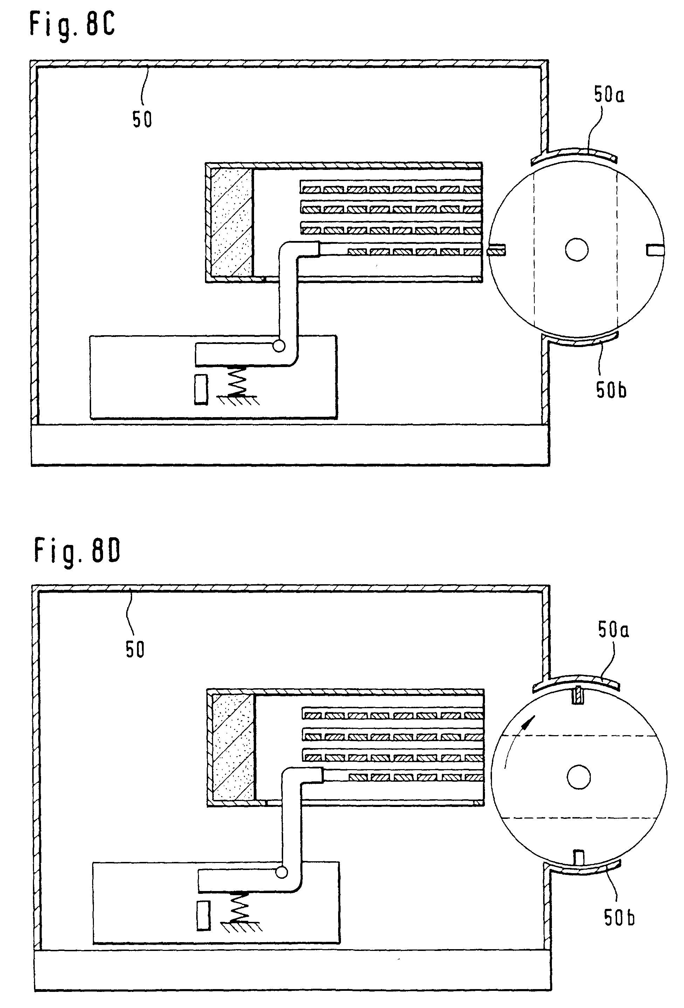

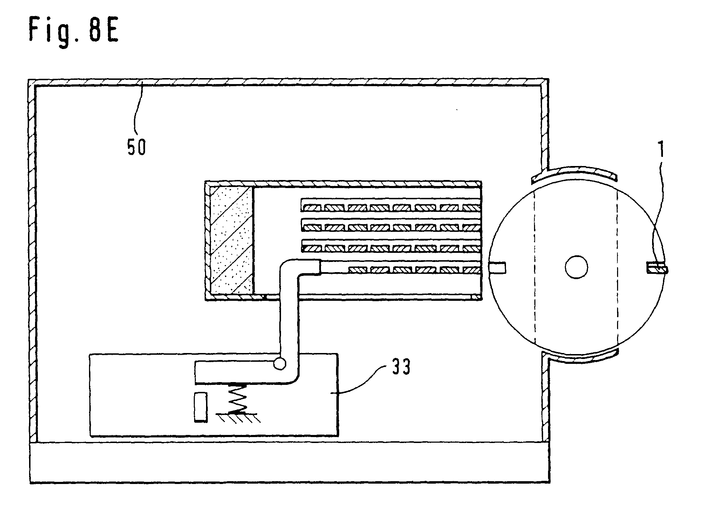

In der Figur 9 ist die Walze (40) in perspektivischer Ansicht dargestellt. In der Walze befindet sich eine Nut (41) die in die Außenumfangsfläche der Walze eingebracht ist und parallel zur Achse der Walze verläuft. In der Nut befindet sich ein Testelement (1). Zur Entnahme des Testelementes weist die Walze vorteilhafterweise einen oder mehrere Schlitze (42) auf, die im wesentlichen senkrecht zur Achse der Walze verlaufen. Die Schlitze (42) erstrecken sich ausgehend von der Oberfläche der Walze in Richtung auf die Walzenachse. Die Form der Schlitze ist vorteilhaft die eines "D". Die Schlitze sind im Bereich der Nut so angebracht, daß sie die Nut hinterschneiden und im Bereich der Schlitze hinter dem Testelement ein Raum zugänglich wird, der zur Entnahme des Testelementes genutzt werden kann.

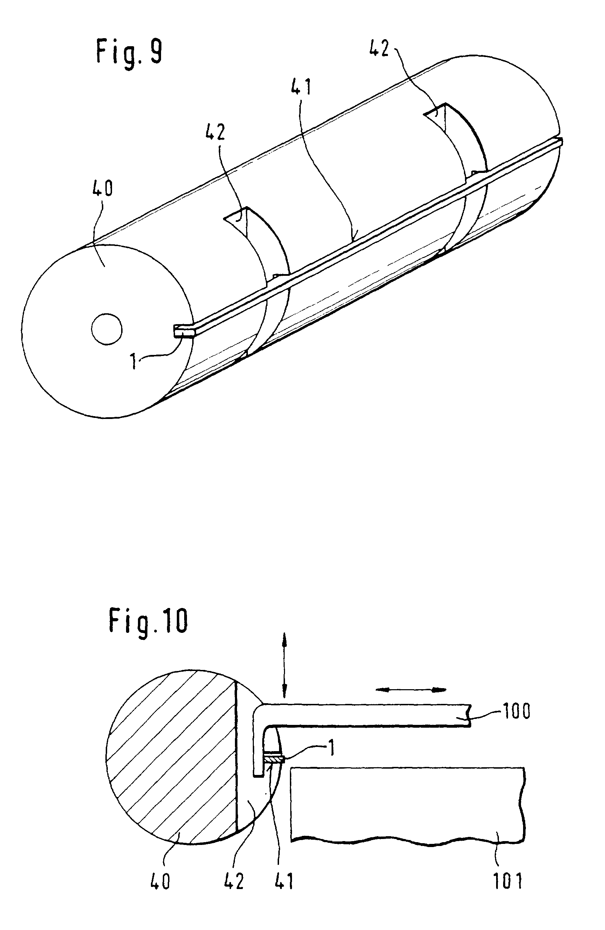

In der Figur 10 ist die in Figur 9 dargestellte Walze im Querschnitt zusammen mit einem Rechen (100) zur Entnahme gezeigt. Der Rechen (100) kann beispielsweise die Form eines Hakens aufweisen und zur Entnahme des Testelementes (1) durch den Schlitz (42) hinter das Testelement geführt werden. Mit einer Ziehbewegung des Rechens kann das Testelement aus der Nut (41) heraus auf eine Unterlage (101) gezogen werden. Von hier aus kann das Testelement weiteren Prozeßschritten zur Durchführung einer Analyse unterzogen werden.

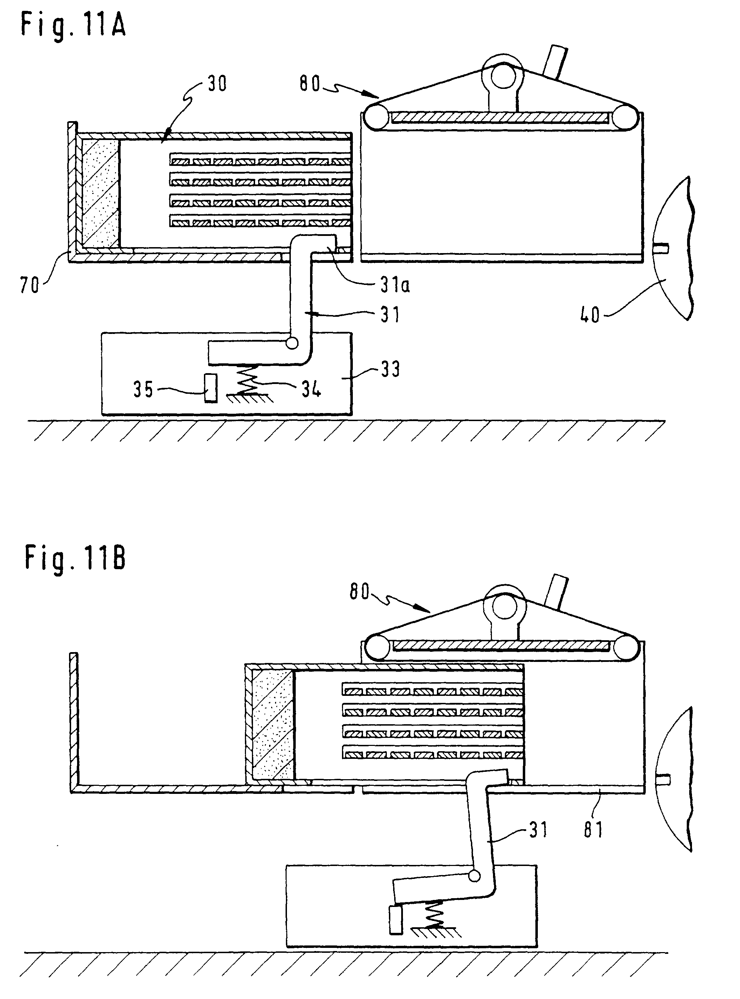

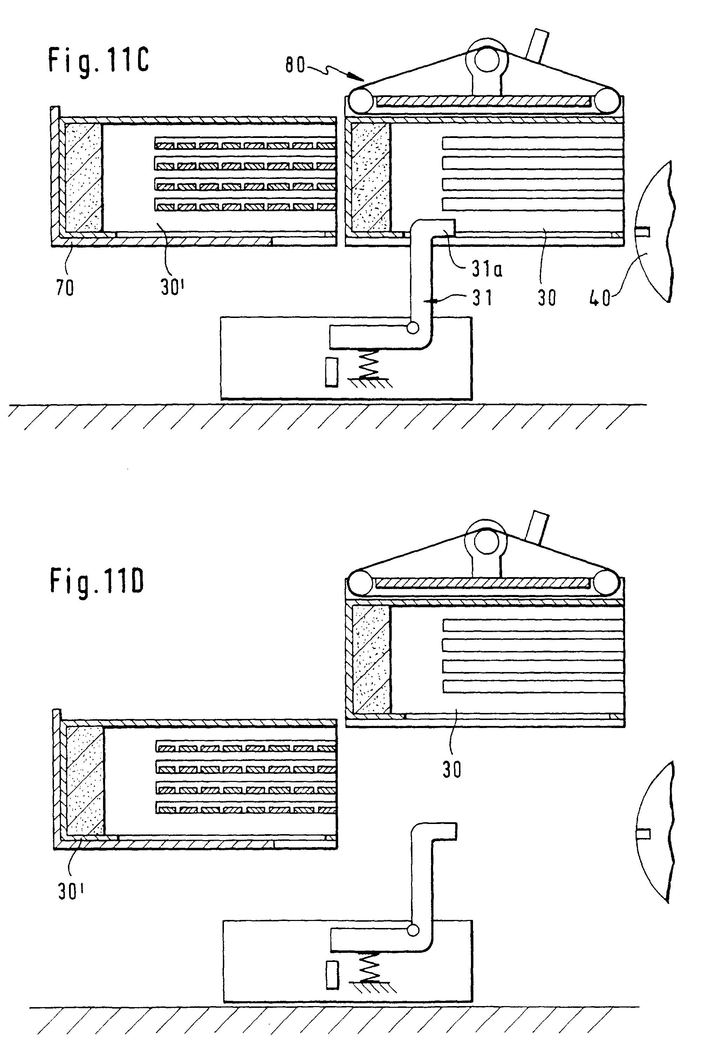

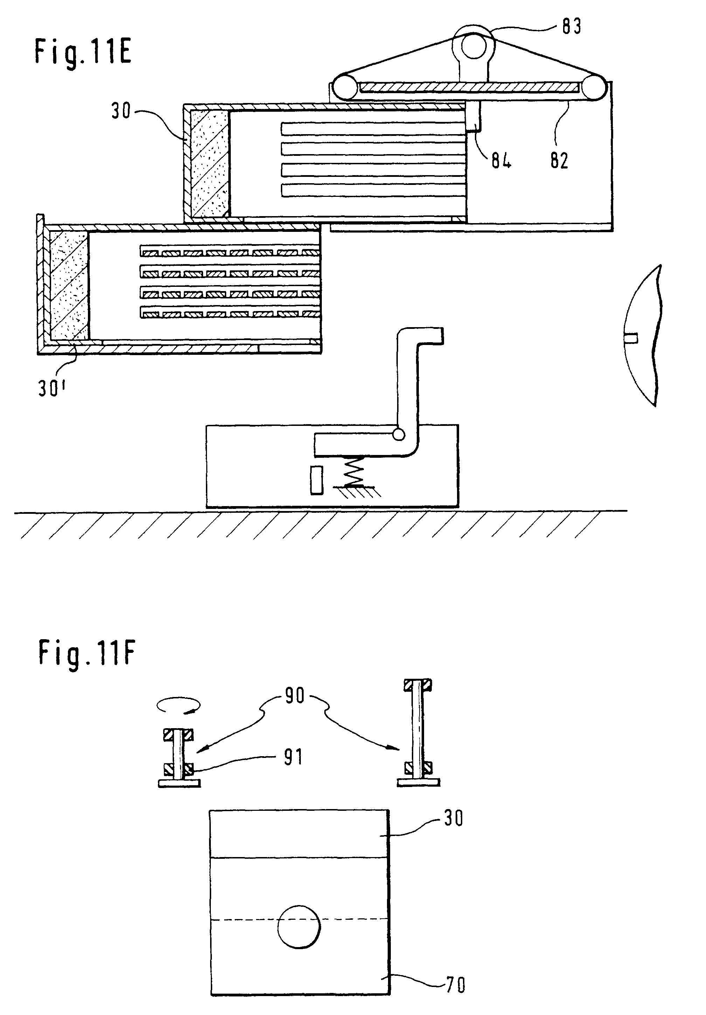

In den Figuren 11A bis F ist ein System zum Zurverfügungstellen von Testelementen dargestellt, das eine Nachfülleinrichtung für Magazine besitzt. Das System gemäß Figur 11 entspricht was die Funktion des Schiebers (31) und der Walze (40) betrifft, im wesentlichen dem in Figur 8 dargestellten System. Aufbauend auf dem System gemäß Figur 8 sind mit dem in Figur 11 dargestellten System jedoch zusätzliche Funktionen möglich. In Figur 11A ist eine Schublade (70) zu erkennen, die herausgezogen und mit einem Magazin (30) befüllt in das System eingeschoben werden kann. Zur Entnahme von Testelementen aus dem Magazin wird das Magazin in eine Entnahmeposition benachbart zur Walze (40) verfahren (analog Figur 8). Hierzuh besitzt das in Figur 11A dargestellte System eine in der Höhe verfahrbare Hebebühne (80), in die ein Magazin aus der Schublade eingeschoben werden kann, wozu eine separate Vorrichtung vorgesehen werden kann. Vorteilhaft ist es jedoch möglich, den Schieber (31) zum Verschieben des Magazins zu verwenden. Hierzu wird der Schlitten (33) so weit nach links verfahren, daß der Schieber (31) von der Unterseite in das in der Schublade (70) befindliche Magazin (30) eingreift. Der Schlitten (33) wird nunmehr in Richtung auf die Walze (40) verfahren, sodaß der Schieber (31) mit seinem vorderen Ende (31a) an einer Kante des Magazins angreift und das Magazin in die Hebebühne hineinzieht, wie dies in Figur 11B dargestellt ist. Zur Aufnahme des Magazins besitzt die Hebebühne (80) eine Bodenfläche (81), durch die das Magazin vor einem Herabfallen bewahrt wird. Die Bodenfläche (81) besitzt eine Ausnehmung, in der der Schieber (31) verfahren werden kann. Wenn ein Magazin vollständig in die Hebebühne (80) eingeschoben ist, so kann die Entnahme von Testelementen analog der für Figur 8 beschriebenen Vorgehensweise erfolgen. Weiterhin ist es möglich, die nunmehr leere Schublade mit einem neuen Magazin (30') zu befüllen, wie dies in Figur 11C dargestellt ist.

Zur Entnahme von Testelementen aus den einzelnen Lagen des Magazins kann das in der Hebebühen befindliche Magazin mit der Hebebühne (80) relativ zu dem Schieber (31) und der Walze (40) in der Höhe verschoben werden. Eine Entnahme erfolgt jeweils aus der zwischen dem vorderen Ende des Schiebers und der Nut der Walze befindlichen Lage.

Nachdem ein in der Hebebühne (80) befindliches Magazin entleert wurde, kann es auf einfache Weise gegen ein neues Magazin ausgetauscht werden. Hierzu verfährt die Hebebühne (80) in eine Höhe, in der sich die Unterkante des Magazins (30) oberhalb der Oberkante des Magazins (30') befindet (siehe Figur 11D). Nunmehr wird ein zur Hebebühne gehörender Mechanismus aktiviert, der das Magazin (30) aus der Hebebühne heraus auf das Magazin (30') verschiebt. Hierzu kann die Hebebühne vorzugsweise einen umlaufenden Riemen (82) besitzten, der über einen Motor (83) angetrieben wird. An dem Riemen ist ein Auswurfnocken (84) befestigt, der bei Bewegung des Riemens das Magazin (30) wie gewünscht verschiebt. Das Magazin (30) kann beispielsweise auf dem Magazin (30') zu liegen kommen und fällt wenn das Magzin (30') entsprechend Figur 11B unter ihm hinweggezogen wird, in die Schublade. Vorzugsweise besitzt das System gemäß Figur 11 jedoch die in Figur 11F beispielhaft dargestellte Haltevorrichtung. Beim Verschieben des Magazins (30) aus der Hebebühne heraus wird das Magazin zunächst in eine Haltevorrichtung eingeschoben. In Figur 11F sind zwei gegenüber angeordnete Rollen (90) dargestellt, zwischen die das Magazin (30) geschoben wird. Die Rollen (90) besitzen vorteilhaft Gummiauflagen (91) oder dergleichen, so daß beim Einschieben das Magazins (30) ein enger Kontakt zwischen den Rollen und dem Magazin hergestellt wird, infolgedessen sich die Rollen beim Einschieben des Magazins drehen und das Magazin nicht unkontrolliert herabfallen kann. Zum Absenken des Magazins in die Schublade (70) werden die Rollen (90) voneinander wegbewegt, so daß sie das Magazin freigeben. In Figur 11F ist bereits die Position der Rollen dargestellt, in der das Magazin freigegeben wird. Durch den beschriebenen Mechanismus ist ein kontrolliertes Absenken entleerter Magazine in die Schublade möglich, ohne daß ein Verklemmen erfolgt.

Das entleerte Magazin, das sich nunmehr in der Schublade befindet, kann vom Benutzer entnommen werden und gegebenenfalls durch eine frisches Magazin ersetzt werden. Somit ist mit diesem System ein kontinuierlicher Betrieb möglich, der lediglich selten eine Aktion des Benutzers benötigt.

### Bezugszeichenliste

- (1): Testelement
- (2): Träger
- (3): Testzone
- (10a): linke Seitenfläche
- (10b): rechte Seitenfläche
- (11): Trockenmittelkammer
- (12): Vorderseite
- (13): Pfeil im Vorschubrichtung
- (14): Ausstoßschieber
- (15): Pfeil in Ausstoßrichtung
- (16a, 16b): Schlitze
- (17): Schieber
- (18a, 18b): Nutenpaar
- (20): einzelner Rahmen
- (21): Rand
- (22): Absatz
- (23): rechteckige Ausdehnung
- (24): Schlitz
- (30): Magazin
- (31): Schieber
- (31a): vorderes Ende des Schiebers
- (31b): hinteres Ende des Schiebers
- (32): Achse
- (33): Schlitten
- (34): Feder
- (40): Sensor
- (40): Walze
- (41): Nut
- (42): Schlitz
- (50): Gehäuse
- (60): Trockenmittel
- (70): Schublade
- (80): Hebebühne
- (81): Boden der Hebebühne
- (82): Riemen
- (83): Motor
- (84): Auswurfnocken
- (90): Rollen
- (91): Gummiauflage
- (100): Rechen
- (101): Unterlage

## Patentansprüche

1. Magazin zur Bevorratung von Testelementen mit einer oder mehreren Testzonen, die nebeneinander auf einem rechteckigen Träger befestigt sind, wobei das Magazin mindestens ein Paar gegenüberliegender Führungsnuten aufweist, in die die Testelemente so eingeschoben sind, daß sie direkt nebeneinander liegen und Kanten benachbarter Träger aneinanderstoßen.

2. Magazin gemäß Anspruch 1, mit zwei oder mehr Paaren gegenüberliegender Führungsnuten, wobei die Paare übereinander angeordnet sind.

3. Magazin gemäß Anspruch 1 oder 2, das Wandungen aufweist, die das Magazin gegenüber dem Außenraum abschließen.

4. Magazin gemäß Anspruch 2, bei dem die Führungsnuten in eine Seitenwand des Magazins integriert sind und das pro Paar von Führungsnuten eine Öffnung besitzt, die in einer Seitenwand angeordnet ist.

5. Magazin gemäß Anspruch 4, bei dem die Öffnung einen rechteckigen Querschnitt besitzt, dessen Höhe um 10 bis 50 % größer ist als die maximale Höhe der Testelemente und dessen Breite um 10 bis 50 % größer ist als die Breite der Testelemente.

6. Magazin gemäß Anspruch 4, bei dem das Magazin eine Stirnfläche besitzt, die im wesentlichen senkrecht zu den Seitenwänden verläuft.

7. Magazin gemäß Anspruch 6, bei dem sich in der Stirnfläche entweder pro Paar von Nuten eine Öffnung befindet, die so angeordnet ist, daß Testelemente, die in den Nuten verschoben werden, durch die Öffnung aus dem Magazin austreten können oder die Stirnfläche offen ist.

8. Magazin gemäß Anspruch 7, bei dem die Öffnung einen rechteckigen Querschnitt besitzt, dessen Breite um 10 bis 30 % größer ist als die maximale Höhe der Testelemente und dessen Länge um 0,5 bis 20 % größer ist als die Teststreifenlänge.

9. Magazin gemäß Anspruch 3, beinhaltend mindestens eine Ausnehmung in einer Wandung, die parallel zu der Ebene von Testelementen verläuft.

10. Magazin gemäß Anspruch 7, beinhaltend Federelemente, die die Testelemente innerhalb der Nut so verschieben, daß ein Testelement gegenüber der Öffnung zu liegen kommt und durch die Öffnung entnommen werden kann.

11. Magazin gemäß Anspruch 1, das in seinem Inneren ein Trockenmittel enthält.

12. Magazin gemäß Anspruch 1, bei dem die Vorderseite des Magazins zumindest teilweise durch eine Siegelfolie verschlossen ist.

13. Magazin gemäß Anspruch 1, bei dem die Vorderseite zumindest teilweise durch eine Siegelfolie und die Rückseite durch eine Trockenmittelkammer verschlossen ist.

14. Magazin gemäß Anspruch 1 oder 13, das aus den folgenden Elementen aufgebaut ist:
- zwei Seitenwände mit integrierten Führungsnuten,
- zwei Wände, die die Seitenwände so miteinander verbinden, daß ein Quader entsteht
- eine Trockenmittelkammer, die an der Rückseite des Magazins angeordnet ist.

15. Magazin gemäß Anspruch 14, bei dem die offene Stirnfläche des Quaders zumindest teilweise durch eine Siegelfolie verschlossen ist.

16. Magazin gemäß Anspruch 14, bei dem die genannten Teile als ein zusammenhängendes Spritzgußteil hergestellt werden und über Filmscharniere miteinander verbunden sind.

17. System zum Zuverfügungstellen von Testelementen beinhaltend
- ein Magazin mit mindestens zwei gegenüberliegenden Führungsnuten, in die Testelemente so eingeschoben sind, daß sie in einer Fläche direkt nebeneinander liegen und Kanten benachbarter Testelemente aneinanderstoßen,
- einen Schieber, der an einem Ende der durch die Testelemente gebildeten Lage angreift und die Testelemente entlang der Nuten in Richtung auf das gegenüberliegende Ende der Lage verschiebt.

18. System gemäß Anspruch 17, bei dem das Magazin eine Wand mit mindestens einer Ausnehmung besitzt, durch die der Schieber in das Magazin eingeführt wird.

19. System gemäß Anspruch 17, bei dem der Schieber durch eine Antriebseinheit angetrieben wird.

20. System gemäß Anspruch 19, bei dem die Antriebseinheit den Schieber in eine Raumrichtung um ein Wegstück abhängig von der Breite der Testelemente verschiebt.

21. System gemäß Anspruch 19 oder 20, bei dem die Antriebseinheit die Eindringtiefe des Schiebers in das Magazin steuert

22. System gemäß Anspruch 17, mit einem zweiten Schieber, der ein Testelement, das sich in einer Entnahmeposition befindet, durch Verschiebung in Längsrichtung des Testelements aus dem Magazin herausbefördert.

23. System gemäß Anspruch 22, bei dem das Magazin in der Entnahmeposition eine erste und eine zweite Öffnung aufweist und der zweite Schieber in die erste Öffnung eindringt und das in der Entnahmeposition befindliche Testelement durch die zweite Öffnung herausschiebt.

24. System zum Zurverfügungstellen von Testelementen gemäß Anspruch 17 mit einer um ihre Achse drehbar gelagerten Walze, in der sich mindestens eine Nut zur Aufnahme eines Testelementes befindet.

25. System gemäß Anspruch 24, bei dem die Walze gegenüber dem Magazin so angeordnet ist, daß ein Testelement, das durch den Schieber aus dem Magazin herausgeschoben wird, in die mindestens eine Nut der Walze eingeschoben wird.

26. System gemäß Anspruch 24 oder 25, bei dem das Magazin in einem im wesentlichen feuchtigkeitsdichten Gehäuse mit einer Ausnehmung angeordnet ist und die Ausnehmung durch die Walze derart verschlossen ist, daß sich die mindestens eine Nut in einer ersten Position der Walze zur Aufnahme eines Testelementes innerhalb des Gehäuses befindet und durch Drehung der Walze um ihre Achse aus dem Gehäuse heraustransportiert werden kann.

27. System gemäß Anspruch 26, bei dem das Gehäuse einen Schutzteil besitzt, der so gegenüber einem Teil der Außenumfangsfläche der Walze angeordnet ist, daß zwischen Walze und Schutzteil ein Spalt gebildet wird, der es ermöglicht ein Testelement, das sich in der mindestens einen Nut befindet, und über die Außenkontur der Walze hinausragt, durch Drehen der Walze durch ihn hindurchzuführen und der eine Kriechstrecke bildet, durch die ein Eindringen von Feuchtigkeit in das Gehäuse reduziert wird.

28. System gemäß einem der Ansprüche 24 bis 27, bei dem die Walze mindestens einen Einschnitt besitzt, der im wesentlichen senkrecht zur Längsachse der Walze im Bereich der mindestens einen Nut verläuft, und der tiefer als die mindestens eine Nut ist, so daß sich im Einschnitt hinter der Nut ein Bereich zum Hintergreifen eines in der Nut befindlichen Testelementes ergibt.

29. System gemäß Anspruch 28 mit einem Rechen, der außerhalb des Gehäuses angeordnet ist und in eine erste Position gebracht werden kann, in der er in den Einschnitt eingreift und ein in der Nut befindliches Testelement hintergreift und der zur Entnahme des Testelementes aus der Nut in eine zweite Position verfahren werden kann.

30. System gemäß Anspruch 17, bei dem der Schieber über eine Achse an einem Schlitten befestigt ist, zu dem er sich in seiner Ruhelage in einer Winkelposition befindet, aus der er herausgedreht wird, wenn der Schlitten verschoben wird und der Schieber durch die Lage von Testelementen einen Widerstand erfährt.

31. System gemäß Anspruch 30 mit einer Detektionseinheit, die ein Herausdrehen des Schiebers aus seiner Ruhelage detektiert.

32. System gemäß Anspruch 17, das eine Aufnahmeposition für ein weiteres Magazin sowie einen Hebemechanismus zum Herausheben eines zumindest teilweise entleerten Magazins aus einer Position zur Entnahme von Testelementen besitzt.

## Claims

1. Magazine for storing test elements with one or several test zones which are attached adjacent to one another on a rectangular support, wherein the magazine has at least a pair of guide grooves located opposite to one another into which the test elements are inserted in such a way that they lie directly adjacent to one another and the edges of adjacent supports abut one another.

2. Magazine as claimed in claim 1, with two or several pairs of opposing guide grooves, wherein the pairs are arranged above one another.

3. Magazine as claimed in claim 1 or 2, wherein it has walls which close the magazine to the external space.

4. Magazine as claimed in claim 2, in which the guide grooves are integrated into a side wall of the magazine and which has one opening per pair of guide grooves which is located in a side wall.

5. Magazine as claimed in claim 4, in which the opening has a rectangular cross-section, its height is 10 to 50 % larger than the maximum height of the test elements and its width is 10 to 50 % larger than the width of the test elements.

6. Magazine as claimed in claim 4, wherein the magazine has a front face which is essentially perpendicular to the side walls.

7. Magazine as claimed in claim 6, wherein there is either one opening per pair of grooves in the front face which is located such that test elements which are moved in the grooves can emerge from the magazine through the opening or the front face is open.

8. Magazine as claimed in claim 7, wherein the opening has a rectangular cross-section, its width is 10 to 30 % larger than the maximum height of the test elements and its length is 0.5 to 20 % larger than the length of the test strips.

9. Magazine as claimed in claim 3 comprising at least one cut-out in a wall which is parallel to the plane of test elements.

10. Magazine as claimed in claim 7 comprising spring elements which move the test elements within the groove so that a test element comes to lie opposite the opening and can be removed through the opening.

11. Magazine as claimed in claim 1, which contains a desiccant in its interior.

12. Magazine as claimed in claim 1, wherein the front side of the magazine is at least partially sealed by a sealing foil.

13. Magazine as claimed in claim 1, wherein the front side is at least partially sealed by a sealing foil and the rear side is sealed by a desiccant chamber.

14. Magazine as claimed in claim 1 or 13, which is composed of the following elements:
- two side walls with integrated guide grooves,
- two walls which link the side walls together in such a way that a cuboid is formed
- a desiccant chamber which is located at the rear side of the magazine.

15. Magazine as claimed in claim 14, wherein the open end face of the cuboid is at least partially closed by a sealing foil.

16. Magazine as claimed in claim 14, wherein the said parts are manufactured as a connected injection moulded part and are joined together by film hinges.

17. System for dispensing test elements which includes
- a magazine with at least two opposite guide grooves into which the test elements are inserted such that they lie directly next to one another in one plane and the edges of adjacent test elements abut one another
- a slide which at one end engages the plane formed by the test elements and moves the test elements along the grooves towards the opposite end of the layer.

18. System as claimed in claim 17, wherein the magazine has a wall with at least one cut-out through which the slide is inserted into the magazine.

19. System as claimed in claim 17, wherein the slide is driven by a drive unit.

20. System as claimed in claim 19, wherein the drive unit moves the slide in one direction in space by an incremental distance depending on the width of the test elements.

21. System as claimed in claim 19 or 20, wherein the drive unit controls the depth of penetration of the slide into the magazine.

22. System as claimed in claim 17, comprising a second slide which transports a test element which is located in a removal position from the magazine by sliding it in the longitudinal direction of the test element.

23. System as claimed in claim 22, wherein the magazine has a first and second opening in the removal position and the second slide enters into the first opening and pushes the test element located in the removal position through the second opening.

24. System for dispensing test elements as claimed in claim 17 comprising a pivoted roller which contains at least one groove for holding a test element.

25. System as claimed in claim 24, wherein the roller is arranged relative to the magazine such that a test element which is pushed out of the magazine by the slide is pushed into the at least one groove of the roller.

26. System as claimed in claim 24 or 25, wherein the magazine is located in an essentially moisture-tight housing with a cut-out and the cut-out is closed by the roller in such a way that at least one groove is located in a first position of the roller for receiving a test element within the housing and can be transported out of the housing by rotating the roller around its axis.

27. System as claimed in claim 26, wherein the housing has a protecting part which is arranged relative to a part of the exterior peripheral surface of the roller such that a gap is formed between the roller and the protecting part which enables a test element which is located in the at least one groove and which juts out above the exterior contour of the roller to be guided through it by turning the roller and which forms a leakage path which reduces penetration of moisture into the housing.

28. System as claimed in one of the claims 24 to 27, wherein the roller has at least one notch which is essentially perpendicular to the longitudinal axis of the roller in the area of the at least one groove and which is deeper than the at least one groove so that a region is formed in the notch behind the groove for gripping behind a test element located in the groove.

29. System as claimed in claim 28 comprising a rake which is located outside of the housing and can be moved into a first position in which it engages in the notch and grips behind a test element located in the groove and which can be moved into a second position in order to remove the test element from the groove.

30. System as claimed in claim 17, wherein the slide is attached via a spindle to a sliding carriage and in its resting position is in an angled position relative thereto from which it is turned away when the sliding carriage is moved and the layer of test elements offers a resistance to the slide.

31. System as claimed in claim 30 comprising a detection unit which detects a rotation of the slide from its resting position.

32. System as claimed in claim 17, which has a holding position for an additional magazine and a lifting mechanism to lift an at least partially empty magazine from a position for removing test elements.

## Revendications

1. Magasin pour le stockage d'éléments d'analyse avec une ou plusieurs zones d'analyse, qui sont fixés les uns à côté des autres sur un support rectangulaire, le magasin étant pourvu d'au moins une paire de rainures de guidage opposées dans lesquelles les éléments d'analyse sont introduits de manière à être posés directement les uns à côtés des autres et de manière à ce que les bords de supports contigus se touchent.

2. Magasin selon la revendication 1, pourvu de deux ou de plusieurs paires de rainures de guidage opposées, les paires étant situées les unes au-dessus des autres.

3. Magasin selon la revendication 1 ou 2, comprenant des parois qui isolent le magasin vis-à-vis de l'espace extérieur.

4. Magasin selon la revendication 2, dans lequel les rainures de guidage sont intégrées dans une paroi latérale du magasin et qui possède, pour chaque paire de rainures de guidage, une ouverture qui est située dans une paroi latérale.

5. Magasin selon la revendication 4, dans lequel l'ouverture a une section transversale rectangulaire dont la hauteur est supérieure de 10 à 50% à la hauteur maximale des éléments d'analyse et dont la largeur est supérieure de 10 à 50% à la largeur des éléments d'analyse.

6. Magasin selon la revendication 4, dans lequel le magasin possède une surface frontale qui s'étend de manière essentiellement perpendiculaire aux parois latérales.

7. Magasin selon la revendication 6, dans lequel, soit la surface frontale comporte, pour chaque paire de rainures, une ouverture qui est située de manière à ce que les éléments d'analyse qui sont déplacés dans les rainures puissent sortir du magasin par l'ouverture, soit la surface frontale est ouverte.

8. Magasin selon la revendication 7, dans lequel l'ouverture a une section transversale rectangulaire dont la largeur est supérieure de 10 à 30% à la hauteur maximale des éléments d'analyse et dont la longueur est supérieure de 0,5 à 20% à la longueur des bandes de test.

9. Magasin selon la revendication 3, comportant au moins un évidement dans une paroi s'étendant parallèlement au plan des éléments d'analyse.

10. Magasin selon la revendication 7, comprenant des éléments élastiques qui déplacent les éléments d'analyse à l'intérieur de la rainure de manière à ce qu'un élément d'analyse vienne se placer face à l'ouverture et puisse être retiré par l'ouverture.

11. Magasin selon la revendication 1, qui contient à l'intérieur un dessiccateur.

12. Magasin selon la revendication 1, dans lequel le côté antérieur du magasin est fermé, au moins partiellement, par une feuille de scellement.

13. Magasin selon la revendication 1, dans lequel le côté antérieur est fermé, au moins partiellement, par une feuille de scellement et le côté postérieur est fermé par une chambre pour un dessiccateur.

14. Magasin selon la revendication 1 ou 13, qui est formé par les éléments suivants :
- deux parois latérales avec des rainures de guidage intégrées,
- deux parois qui relient les parois latérales l'une à l'autre de manière à former un carré,
- une chambre pour un dessiccateur, qui est située sur le côté postérieur du magasin.

15. Magasin selon la revendication 14, dans lequel la surface frontale ouverte du carré est fermée, au moins partiellement, par une feuille de scellement.

16. Magasin selon la revendication 14, dans lequel les parties mentionnées sont fabriquées sous la forme d'une pièce moulée par injection continue et sont reliées les unes aux autres par le biais de charnières à film.

17. Système pour la mise à disposition d'éléments d'analyse, comprenant :
- un magasin pourvu d'au moins deux rainures de guidage opposées dans lesquelles les éléments d'analyse sont introduits de manière à être posés directement les uns à côté des autres dans une surface et de manière à ce que les bords d'éléments d'analyse contigus se touchent,
- un élément coulissant qui s'emboîte à une extrémité de la couche formée par les éléments d'analyse et qui déplace les éléments d'analyse le long des rainures en direction de l'extrémité opposée de la couche.

18. Système selon la revendication 17, dans lequel le magasin possède une paroi comportant au moins un évidement à travers lequel l'élément coulissant est introduit dans le magasin.

19. Système selon la revendication 17, dans lequel l'élément coulissant est commandé par une unité d'actionnement.

20. Système selon la revendication 19, dans lequel l'unité d'actionnement déplace l'élément coulissant dans une direction de l'espace sur une distance qui dépend de la largeur des éléments d'analyse.

21. Système selon la revendication 19 ou 20, dans lequel l'unité d'actionnement commande la profondeur de pénétration de l'élément coulissant dans le magasin.

22. Système selon la revendication 17, comprenant un second élément coulissant qui transporte à l'extérieur du magasin un élément d'analyse se trouvant dans une position de retrait en déplaçant cet élément d'analyse dans sa direction longitudinale.

23. Système selon la revendication 22, dans lequel le magasin comporte, dans la position de retrait, une première et une seconde ouvertures et le second élément, coulissant pénètre dans la première ouverture et pousse à l'extérieur, à travers la seconde ouverture, l'élément d'analyse se trouvant dans la position de retrait.

24. Système pour la mise à disposition d'éléments d'analyse selon la revendication 17, comprenant un cylindre monté de manière rotative autour de son axe et pourvu d'au moins une rainure destinée à recevoir un élément d'analyse.

25. Système selon la revendication 24, dans lequel le cylindre est situé en face du magasin de manière à ce qu'un élément d'analyse poussé hors du magasin par l'élément coulissant soit introduit dans la - au moins une - rainure du cylindre.

26. Système selon la revendication 24 ou 25, dans lequel le magasin est situé dans un boîtier essentiellement étanche à l'humidité et pourvu d'un évidement, et l'évidement est fermé par le cylindre de manière à ce que, dans une première position du cylindre, la - au moins une - rainure se trouve à l'intérieur du boîtier pour recevoir un élément d'analyse, et à ce que la rotation du cylindre autour de son axe puisse la transporter à l'extérieur du boîtier.

27. Système selon la revendication 26, dans lequel le boîtier possède un élément de protection qui est situé en face d'une partie de la surface circonférentielle extérieure du cylindre de manière à former, entre le cylindre et l'élément de protection, une fente qui permet à un élément d'analyse se trouvant dans la - au moins une - rainure et faisant saillie sur le contour extérieur du cylindre de passer à travers elle, en faisant tourner le cylindre, et qui forme une distance de cheminement grâce à laquelle on réduit la pénétration de l'humidité dans le boîtier.

28. Système selon l'une des revendications 24 à 27, dans lequel le cylindre est pourvu d'au moins une encoche qui s'étend de manière essentiellement perpendiculaire à l'axe longitudinal du cylindre, dans la zone de la - au moins une - rainure, et qui est plus profonde que la - au moins une - rainure, de manière à obtenir, dans l'encoche derrière la rainure, une zone permettant de saisir l'arrière d'un élément d'analyse se trouvant dans la rainure.

29. Système selon la revendication 28, comprenant un râteau qui est situé à l'extérieur du boîtier et peut être amené dans une première position dans laquelle il s'enclenche dans l'encoche et saisit l'arrière d'un élément d'analyse se trouvant dans la rainure, et qui peut être déplacé dans une seconde position pour que l'élément d'analyse soit retiré de la rainure.

30. Système selon la revendication 17, dans lequel l'élément coulissant est fixé à un chariot par le biais d'un axe, chariot par rapport auquel, dans sa position de repos, il se trouve dans une position angulaire qu'une rotation peut lui faire quitter lorsque le chariot est déplacé et que l'élément coulissant subit une résistance de la couche des éléments d'analyse.

31. Système selon la revendication 30, comprenant une unité de détection qui détecte lorsqu'une rotation fait sortir l'élément coulissant de sa position de repos.

32. Système selon la revendication 17, qui possède une position de réception pour un autre magasin ainsi qu'un mécanisme de levage pour faire sortir d'une position de retrait des éléments d'analyse, un magasin au moins partiellement vidé.
